# EUROPEAN PATENT APPLICATION

(11) **EP 1 348 440 A1**
(43) Date of publication of application: **01.10.2003**
(21) Application number: 02076219.1
(22) Date of filing: 28.03.2002
(51) Int. Cl.: A61K 38/08, A61P 9/10

(54) **Use of angiotensin 1-7 for enhancing cardiac function**

(71) Applicant: Citeq B.V., 9726 GN Groningen (NL)
(72) Inventor: Roks, Antonius Jacobus Marinus, 9731 CH Groningen (NL); Henning, Robert Henk, 9919 AJ Loppersum (NL); Van Gilst, Wiekert Hendrikus, 9752 CA Haren (NL); Van der Graaf, Adrianus Cornelis, 9712 BP Groningen (NL)
(74) Representative: Prins, Adrianus Willem

(57) **Abstract**

The invention provides a use of angiotensin-(1-7), or a functional part, derivative and/or analogue thereof, for the preparation of a medicament for enhancing cardiac function. For instance, left ventricular diastolic pressure, coronary flow, endothelial function and/or myocyte hypertrophy can be enhanced by a medicament of the invention. By enhancing cardiac function, the development of heart failure can be attenuated or prevented.

The invention further comprises a pharmaceutical composition comprising angiotensin-(1-7), or a functional part, derivative and/or analogue thereof, and a carrier. A method for treating an animal suffering from, or at risk of suffering from, heart failure comprising administering to said animal a pharmaceutical composition of the present invention is also provided herein.

## Description

The invention relates to the field of medicine. More specifically, the invention relates to treatment of cardiac malfunction.

Cardiovascular disorders form a major cause of mortality in the Western world. Among other things, cardiovascular disorders can involve angina pectoris, myocardial ischemia and heart failure.

Cardiac tissue contains roughly two compartments consisting of cardio-myocytes and non-myocytes, respectively. The cardio-myocytes are highly differentiated cells which have lost the ability to divide, and can adapt only by enlargement, so-called hypertrophy. The non-myocyte compartment consists of cells like fibroblasts, macrophages, vascular smooth muscle cells, vascular endothelial cells, endocardial cells and of an extracellular matrix. Enlargement of the non-myocyte compartment can be achieved by cell division and matrix deposition. Physiological enlargement during normal development and growth, and in response to intense exercise is characterized by an equal increase in both compartments. As a result total myocardial contractility is increased. In contrast, myocardial adaptation in response to pressure/volume overload or myocardial infarction characteristically disturbs normal myocardial architecture, resulting in a relative increase of extracellular matrix and a decrease in capillary density. The relative deficit of capillaries in turn is the trigger for development of ischemia, which leads to deterioration of cardiac function on the long-term.

Heart failure can occur as a result of deteriorated cardiac function. It can follow immediately after a problem or injury affects the heart, but more commonly develops months or years later. Heart failure is a progressive disorder which can begin with any number of culprits and deteriorate if left untreated. The response of the body to heart failure is not clearly defined. Many of the known responses may be reactions to treatment rather than due to heart failure itself. This is particularly true for the renin-angiotensinaldosterone system. Although the natural history of these responses is not known, it seems probable that the first system to be activated is the sympathetic nervous system and only much later other systems begin to interact. Less well understood are the local factors in the peripheral circulation which increase the resistance to blood flow, particularly in exercising skeletal muscle, but there is almost no clue concerning the mechanism involved. Essentially, there are two forms of heart failure. Acute heart failure appears suddenly and requires immediate treatment. In the other, chronic form, patients develop slowly fluid overload over many weeks, months or even years and will often not seek medical attention in this phase. Limited information is available on the responses which occur in this early period of untreated chronic heart failure, although there is evidence of sympathetic stimulation. The pathophysiology of heart failure is best understood in terms of two separate components:
1. Primary myocardial abnormalities, which lead ultimately to pump dysfunction.
2. Decompensation of compensatory mechanisms leading to congestive heart failure.
Cardiac failure can be modeled in terms of responses to a large number of cardiovascular diseases that lead to abnormalities of pump function. These can be divided into:
1. Disorders which impose a sustained increase in volume or pressure load on the heart, such as augmented systolic blood pressure or an increased diastolic volume.
2. Disorders which cause a loss of myocardium and which leaves the remainder of the ventricle to perform an increased workload. Such loss of myocardium may be segmental, as occurs in acute myocardial infarction, or focal and diffuse, as occurs in the various cardiomyopathies.
In all these circumstances the response of the myocardium is initially to undergo hypertrophy as a compensatory mechanism.⁷ The stimuli leading to hypertrophy are not known but wall tension is likely to be one factor. Furthermore, the components of the compensatory mechanisms, such as angiotensin II and catecholamines are potential growth factors inducing hypertrophy. In the first of the above mentioned categories all the myocytes are involved; in the second, of necessity, only those myocytes which survive can respond. Hypertrophy is the antecedent of clinically expressed chronic heart failure.

Hypertrophy may be viewed as a compensatory mechanism occurring in heart failure of any aetiology. Hypertrophy resulting from chronic pressure or volume overloading is not easily reversible and can become pathological. On the other hand, left ventricular hypertrophy is not an all-or-nothing phenomenon; physiological and pathological hypertrophy are different points in a continuous spectrum. Persistence of the stimuli for hypertrophy is the important factor which may invoke a series of secondary and irreversible changes in the myocardium.

The increase in ventricular mass that occurs in early hypertrophy is due to an increase in myocyte size. This enlargement of the myocardial cell may be viewed initially as an adaptive mechanism that is associated with alterations in the biochemistry, electrophysiology, and resultant mechanical contraction of the myocardium. As the degree of hypertrophy increases and left ventricular function decreases, there is a concomitant decrease in the myofibrillar content of individual myocytes. The mitochondrial mass is reduced but to a lesser extent. Therefore, despite what may be seen clinically as a major increase in left ventricular mass, the myofibrillar mass on which systolic contraction depends may not have increased to an equivalent degree. There is a close correlation between reduced volume density of myofibrils within the myocyte and low ejection fraction. The latter stages of hypertrophy also lead to synthesis of different forms and proportions of the isoenzymes of myosin which may have lower ATPase activity than the normal situation. In physiological hypertrophy the myosin ATPase activity is said to be normal or elevated in contrast to the decreased ATPase activity of the latter stages of pathophysiological hypertrophy. Furthermore, fibrous tissue may develop as a diffuse increase in the interstitial tissues and may increase to surround and isolate separate myocytes. This insulation of myocytes by collagen may be important in creating re-entry paths and may account for the increase in ventricular arrhythmias found in ventricular hypertrophy from any cause. The fibrosis in hypertrophy may also be very important in altering the properties of the myocardium in relaxation and diastolic filling.

Concomitantly, there is a slowing of the biochemical pumps responsible for calcium sequestration in the cell. Prolongation of electrical activity occurs at the same time. These phenomena are associated with mechanical alterations of the myocardial contraction, which include slowing of the rate of contraction, prolongation of the time to peak tension development, and a delay in relaxation. When thickening of the ventricular wall and variable amounts of fibrosis supervene, limitations to ventricular filling results in so-called "diastolic dysfunction". Diastolic dysfunction is amplified by the earlier mentioned tachycardia, which limits the duration of ventricular filling. Ultimately, the force of myocardial contraction become reduced as the processes that associate cell loss and excessive hypertrophy progress. Ventricular wall remodeling occurs with augmentation of diastolic function so that ejection fraction decreases. This corresponds to so-called "systolic dysfunction". Although this distinction between systolic and diastolic dysfunction has only recently been appreciated, the concept was already described more than 65 years ago.

Initially, the alterations in ventricular performance may or may not be associated with derangements of the peripheral circulation. However, as these derangements develop, the syndrome of congestive heart failure may ensue.

Congestive heart failure is characterised by a flattening of the Frank Starling curve and a reduced left ventricular ejection fraction. At the same time, depressed cardiac performance tends to be associated with increases in peripheral arterial resistance so that the cardiac output response to exercise is severely limited. This results in fatigue, limitations of exercise performance, and at a later stage, anorexia due to limitation of gastrointestinal blood flow and mental confusion. Such effects are amplified by the neurohumoral responses that lead to increased sympathetic tone and activation of the renin-angiotensin system (RAS). Activation of the renin-angiotensin system with production of angiotensin II leads to peripheral vascular vasoconstriction but also to aldosterone release by the adrenals, which in turn tends to augment sodium accumulation. These latter effects result in both central and peripheral fluid retention and edema. All these sequelae together form the so-called 'vicious circle of heart failure' ; breaking the spiral can alter the prognosis.

The RAS is being considered as one of the most important regulatory systems for cardiovascular homeostasis. It plays a central role in blood pressure regulation, and in growth processes in the vessel wall as well as the myocardium. The key enzyme, the angiotensin converting enzyme (ACE), which is abundantly present on endothelial cells, activates angiotensin II (Ang II) and inactivates bradykinin (BK). Ang II, which is formed from angiotensin I (Ang I) by ACE, is a vasoconstrictor and growth stimulator when acting on the AT1 receptor while BK is a potent vasodilator. BK is degraded by ACE through sequential removal of the dipeptides Phe-Arg and Ser-Pro from the C-terminal end of the decapeptide. In addition to their inhibitory effect on Ang II formation, accumulation (and potentiation) of endogenous BK may be another mechanism by which ACE-inhibitors exert their effects³.

The reduction in pump function (myocardial failure) and the ultimate development of congestive heart failure are commonly dissociated in time, and this fact has important pathophysiologic and moreover therapeutic implications. Probably activation of compensatory mechanisms are playing a key role in the transition from the asymptomatic to the symptomatic state. During primary cardiac failure, a series of compensatory mechanisms is activated to preserve central blood pressure and cardiac output to organs with autoregulatory control of blood flow, namely the brain and the heart. Compensatory responses do not occur by chance; they are similar to those which occur during exercise and during haemorrhage. Heart failure might evoke the same ancient evolutionary responses which evolved in primitive man to deal with fight or flight situations in order to maintain or increase blood pressure. These reflexes are activated as man develops coronary artery disease and heart failure while surviving beyond his reproductive years. In the short term, these compensatory response are as appropriate to the situation as they are in acute haemorrhage or exercise. Nevertheless, the response seen in heart failure resembles a theoretical situation of continuous exercise, which is then inappropriate and harmful in the long term. These compensatory mechanisms and the ensuing induction of counter-regulatory processes, like among many others the increased production of atrial natriuretic peptide, that antagonizes peripheral constriction are capable of supporting cardiac function for a variable period of time, during which time the patient may experience no or only few symptoms. However, at some point in the history of heart failure these mechanisms lose their ability to maintain cardiac function in a compensated state; within five years of diagnosis most subjects with heart failure will be dead due to causes related to progressive pump dysfunction.

There are three potential explanations of why compensatory mechanisms may eventually cease to support the failing heart:
1. The process that initially damaged the myocardium may continue to an extent that exceeds the ability of compensatory mechanisms to stabilize function. For example, the heart failure that accompanies abnormal loading conditions and increased wall stress, as in hypertension and valvular heart disease, may proceed and lead to progressive damage, as may the heart failure that accompanies multiple myocardial infarctions.
2. The compensatory mechanisms may eventually become 'exhausted' and lose their full effect. There is good evidence that the exhaustion of compensatory mechanisms accounts for some of the progressive deterioration in function that is part of the natural history of heart failure. For example, progressive heart failure is accompanied by progressive loss of the beta-adrenergic support mechanism that is the primary means of increasing cardiac contractility in response to the increased demand of stress or exercise. This loss of support occurs by regulatory changes in at least three different constituents in the receptor-G-protein-adenylate cyclase complex (RGC). The most dramatic regulatory change is betas-receptor downregulation, which accounts for most of the beta-RGC desensitization in heart failure. A less important alteration is mild uncoupling of myocardial beta₂-adrenergic receptors, which comprise 30-40% of the ventricular myocardial receptors in the failing human heart. The final regulatory change is upregulation of G₁ₐₗₚₕₐ, which may be responsible for beta₂-receptor uncoupling, and which may also mediate tonic inhibition of the RGC complex by occupying the Gᵢ-coupled receptors.
3. The compensatory mechanisms may somehow injure the myocardium, resulting in secondary damage to the contractile apparatus.

With an activated renin-angiotensin system most of the damage is probably indirect, via increased systolic and diastolic wall stress produced by angiotensin II-induced vasoconstriction and aldosterone-related elevation in left ventricular end-diastolic pressure. However, it is also possible that the renin-angiotensin system, particulary the local renin-angiotensin system, may mediate a direct form of cardiac damage.

The beneficial effects of ACE-inhibitors on hypertrophied myocardium have been described extensively in animal and in human studies. Treatment with ACE-inhibitors not only reduces symptoms, but also improves survival in heart failure patients. Ang II is a potent growth factor for myocytes, fibroblasts, and vascular smooth muscle cell (VSMC). On a cellular level multiple mechanisms play a role. Next to oncogenes and cyclins⁵, interference with cell cycle regulating homeobox genes may be important. Ang II promotes unwanted VSMC proliferation by downregulation of cell cycle arresting genes such as the growth arrest homeobox (gax)⁶.

The effect of BK on cell proliferation is less well described. It has been suggested that BK reduces fibroblast and VSMC proliferation by a prostaglandin- and NO-dependent mechanism. Given all the above, therefore, it is not surprising that upregulation of (cardiac) ACE activity as found after myocardial infarction contributes to unfavorable remodeling of the myocardium: cardiomyocyte hypertrophy, increased matrix, and relative deficit of neovascularisation or angiogenesis.

In conclusion, one of the most successful pharmacotherapeutic interventions in patients, as well as in experimental models of heart failure, currently consists of ACE-inhibitor therapy which is developed to suppress the formation of angiotensin II.^{1,2} Angiotensin converting enzyme inhibitors were able to reduce mortality up to 50 % in NYHA IV patients. However, when the contribution of two decades of development is translated into absolute gain in life-expectancy then the result is little less than one year. Furthermore, in the last two years several newer developments had to be stopped due to inefficacy or even adverse outcome. Recent examples are the disappointing results with the endothelin antagonist and vasopeptidase inhibitors. This indicates that conventional therapy within its present pathophysiological framework has reached a ceiling. Despite promising results, cardiac disorders still cannot always be cured.

The present invention provides additional methods and means to enhance cardiac function. It has been found that heptapeptide angiotensin-(1-7), or a functional part, derivative and/or analogue thereof is very suitable for enhancing cardiac function. Angiotensin-(1-7) (Ang-(1-7)) is a biologically active metabolite of Ang I and Ang II that is formed through cleavage of endopeptidases and is inactivated by ACE.⁴ Under normal conditions, tissue and plasma levels of Ang-(1-7) are similar to those of Ang II.

The invention therefore provides a use of angiotensin-(1-7), or a functional part, derivative and/or analogue thereof, for the preparation of a medicament for enhancing cardiac function. By enhancing cardiac function is meant that at least one aspect of cardiac function is improved. This can also mean that at least one aspect of cardiac function is less deteriorated as compared to said at least one aspect of cardiac function without treatment with angiotensin-(1-7), or a functional part, derivative and/or analogue thereof. Said aspect can for instance comprise left ventricular diastolic pressure, coronary flow, endothelial function (such as aortic endothelial function and/or coronary endothelial function) and/or myocyte hypertrophy. Enhancing said cardiac function can treat a cardiac disorder. Additionally, enhancing said cardiac function can attenuate and/or prevent the occurrence of an (additional) cardiac disorder. For instance the development of heart failure after myocardial infarction can be attenuated or prevented by using angiotensin-(1-7) or a functional part, derivative and/or analogue thereof as a medicament. Although we do not wish to be bound by theory, it is believed that Ang- (1-7) antagonizes the vasoconstrictive effects of Ang II in various species,⁸ and is a vasodilator in canine and porcine coronary arteries,^{9,10} either by blocking the AT1-receptor¹¹ or by releasing nitric oxide and vasodilating prostaglandins via an as yet unidentified receptor.^{10,12,13}

Angiotensin-(1-7) based therapy goes beyond the limits that are involved with conventional therapy. As an endogenous peptide angiotensin-(1-7) has a favourable safety profile and a very broad therapeutic range. Furthermore, evidence is accumulating that angiotensin-(1-7) is the endogenous modulator of an activated renin-angiotensin system. All the previous work with ACE-inhibitors has identified the renin-angiotensin system as the most important pathophysiological mechanism for the progression of heart failure. Angiotensin-(1-7) based therapy offers a treatment with a desired risk/benefit ratio.

A functional part of angiotensin-(1-7) is defined as a part which has the same kind of properties in kind, not necessarily in amount. Said properties for instance comprise the capability of angiotensin-(1-7) to enhance cardiac function. A functional derivative of angiotensin-(1-7) is defined as an angiotensin-(1-7)-derived peptide which has been altered such that the properties of said molecule are essentially the same in kind, not necessarily in amount. A derivative can be provided in many ways, for instance through conservative amino acid substitution. A derivative can also be provided by deletion and/or addition of one or more amino acid residues. A functional derivative can also comprise an angiotensin-(1-7) peptide or angiotensin-(1-7)-derived peptide coupled to another molecule. Such derivative for instance comprises a fusion-protein.

A person skilled in the art is well able to generate analogous compounds of angiotensin-(1-7). This can for instance be done through screening of a peptide library or phage display library. Such an analogue has essentially the same properties of angiotensin-(1-7) in kind, not necessarily in amount. An analogue of angiotensin-(1-7) can also comprise a peptidomimetic and/or a non-peptidic molecule mimicking the capability of angiotensin-(1-7) to enhance cardiac function.

In one aspect the invention provides a pharmaceutical composition comprising angiotensin-(1-7), or a functional part, derivative and/or analogue thereof, and a carrier. Said pharmaceutical composition is particularly suitable for administration to an animal in order to improve the cardiac function of said animal. Said animal preferably comprises a human. A use of angiotensin-(1-7), or a functional part, derivative and/or analogue thereof, for enhancing cardiac function is also herewith provided. Preferably, said cardiac function comprises left ventricular diastolic pressure, coronary flow, endothelial function and/or myocyte hypertrophy. More preferably, said endothelial function comprises aortic endothelial function and/or coronary endothelial function.

Angiotensin-(1-7) or a functional part, derivative and/or analogue thereof can be used for the preparation of a medicament, for instance by mixing said angiotensin-(1-7) or functional part, derivative and/or analogue thereof with a suitable carrier. Said medicament can be administered to an animal orally, as a suppository, by infusion, etc. Additives may be added to said medicament, for instance in order to facilitate administration and/or in order to enhance stability of said medicament.

Furthermore, said angiotensin-(1-7) or functional part, derivative and/or analogue thereof can be used as a medicament for enhancing cardiac function by way of gene therapy. In that case, a nucleic acid encoding angiotensin-(1-7) or a functional part, derivative and/or analogue thereof can be administered to an animal, for instance with aid of a gene delivery vehicle. Said nucleic acid can be incorporated into the genome of said animal, and/or can be present transiently in said animal. Preferably transcription and/or translation of said nucleic acid is controlled by a signal, like for instance by a sequence responsive to exogenous compounds or responsive to increased stimulation of endogenous hormonal systems activated in cardiac disease, such as the RAS, natriuretic peptide system or the sympathetic system. Transcription and translation of said nucleic acid inside said animal results in the generation of angiotensin-(1-7) or a functional part, derivative and/or analogue thereof, which enhances the cardiac function of said animal. As used herein, an animal can comprise a human and/or a non-human animal.

In one aspect of the invention, treatment involving angiotensin-(1-7) in a DNA based strategy comprises a treatment that is targeted to specific organs only, preferably the heart. Locally even higher levels of angiotensin-(1-7) can be produced leading to resetting of the local derailed system. This treatment can be repeated with for instance yearly intervals. In one embodiment of the invention, an angiotensin-(1-7) gene construct leads to conditional expression. The promoter of said construct reacts on the increase of neurohumoral levels indicative for a deterioration of heart failure.

Of course, a person skilled in the art is well capable of choosing alternative ways for using angiotensin-(1-7) or a functional part, derivative and/or analogue thereof as a medicament for enhancing cardiac function. Likewise, a person skilled in the art is well capable of performing alternative methods for using angiotensin-(1-7) or a functional part, derivative and/or analogue thereof for the preparation of a medicament.

One cardiac disorder which can be counteracted by enhancing cardiac function with angiotensin-(1-7) or a functional part, derivative and/or analogue thereof comprises heart failure. In the examples it is shown that angiotensin-(1-7) can be used for attenuating the development of heart failure.

One embodiment of the invention therefore provides a method for treating an animal suffering from, or at risk of suffering from, heart failure comprising administering to said animal a pharmaceutical composition of the invention. In a further embodiment a method of the invention is provided wherein said heart failure, or risk of heart failure, occurs after a myocardial infarction. Preferably, said pharmaceutical composition is administered to said animal by infusion, more preferably by continuous intravenous infusion. However, other ways of administration are possible, as explained above.

Yet another embodiment of the invention provides a use of angiotensin-(1-7), or a functional part, derivative and/or analogue thereof, for treatment of an animal suffering from, or at risk of suffering from, heart failure. Said heart failure, or risk of heart failure, may occur after a myocardial infarction, although this is not necessary.

The following examples are meant to explain the present invention in more detail. They do not limit the invention in any way. A person skilled in the art is well capable of performing alternative methods which are in the scope of the present invention.

### EXAMPLES

### Experimental Design

The Animal Research Committee of the University of Groningen approved this study. Left coronary artery ligations were performed in 39 male Sprague-Dawley rats weighing 250 to 300 g (Harlan; Zeist, the Netherlands).¹⁴ Perioperative mortality was 49%. Two weeks after induction of MI, rats were randomly allocated to intravenous infusion of either Ang-(1-7) (24 µg/kg per hour; n =10) or saline (n =10) by osmotic minipumps (Alzet 2004). Sham-operated controls (n =10) received saline. After 8 weeks of treatment, hemodynamic studies were performed under isoflurane anesthesia with a microtip pressure transducer,¹⁵ coronary flow was measured in a Langendorff setup,¹⁴ endothelial function was tested in isolated aortic rings,¹⁴ and plasma Ang-(1-7) levels were measured by radioimmunoassay.¹⁶

### Histology

Midventricular slices were processed for histochemical analysis. Infarct size was determined on picrosirius red/fast green-stained sections and was expressed as the percentage of scar length of total left ventricular circumference.¹⁴ Rats with infarcts smaller than 20% were excluded (n =3 for Ang-(1-7) and n =1 for saline-treated rats). Capillary density was determined on sections stained with biotinelabeled *Griffonia simplicifolia* lectin I (GSL-I) and hematoxylin and was expressed as the number of capillaries per mm². Myocyte cross-sectional area was measured on hematoxylin/eosin-stained sections.

### Statistical Analysis

Data are presented as mean ± SEM. Statistical analysis between the groups was performed by 1-way ANOVA followed by Bonferroni's *t* test. Differences in dose-response curves were tested by ANOVA for repeated measures with Greenhouse-Geisser correction for asphericity. Differences were considered significant at *P* < 0.05.

### Results

### General Characteristics

General parameters at the end of treatment are shown in Table 1. There was no difference in body weight among the 3 groups. Infarct size did not differ between the Ang-(1-7) and saline-treated group, with an average of 33%. Left ventricular weight to body weight ratios were equally increased in both MI groups compared with sham-operated controls (17%, *P* <0.05). Myocyte cross-sectional area was significantly increased after infarction, and the increase was attenuated to a nonsignificant level by Ang-(1-7). Capillary density was diminished in infarcted rats, but did not differ between the Ang-(1-7) and saline-treated groups. To confirm delivery of the peptide, Ang-(1-7) plasma levels were measured at the end of treatment. Intravenous infusion of Ang-(1-7) increased plasma levels of the peptide 40-fold compared with MI controls to 917.8±194.1 pmol/L (Table 1).

**Table 1.**

| **General characteristics after 8 weeks of treatment** | | | |
|---|---|---|---|
| | **sham** | **MI control** | **MI Ang-(1-7)** |
| BW (g) | 432.9 ± 6.8 | 418.0 ± 6.1 | 414.0 ±9.0 |
| infarct size (%) | - | 35.5 ± 2.2 | 29.6 ± 3.3 |
| LVW/BW (mg/g) | 2.88 ± 0.08 | 3.46 ± 0.14* | 3.42 ± 0.10* |
| [Ang-(1-7)] (pmol/l) | 9.9 ± 1.9 | 22.9 ± 7.8 | 917.8 ± 194.1* ^{†} |
| myocyte cross-sectional area | 341 ± 17 | 456 ± 25* | 409 ± 21 |
| capillary density (N/mm²) | 3104 ±142 | 2531 ± 179* | 2578 ± 176* |

| | | | |
|---|---|---|---|
| Results are expressed as mean ± SEM, BW: body weight, LVW/BW: left ventricular weight body weight ratio, [Ang-(1-7)]: plasma concentration of angiotensin-(1-7); * p < 0.05 vs. sham, ^{†}p < 0.05 vs. MI control. | | | |

### Hemodynamics

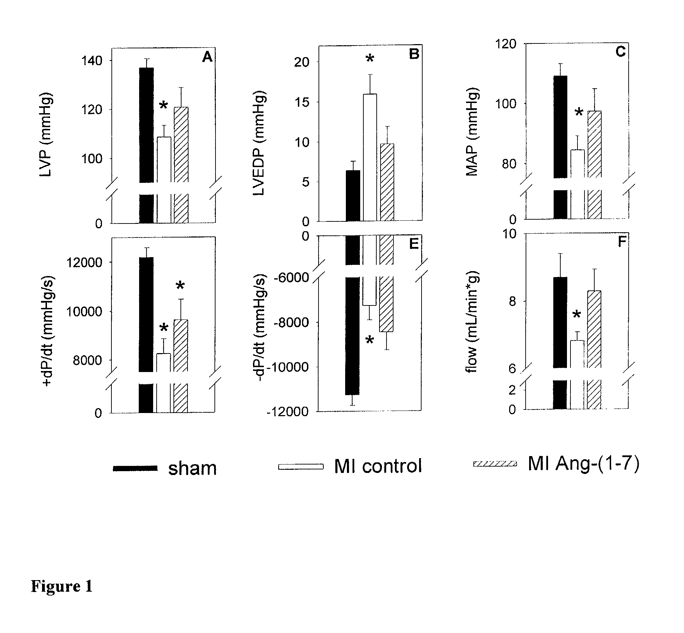

After 8 weeks of treatment, cardiac function was measured *in vivo* in anesthetized rats. As expected, cardiac function was significantly impaired in untreated MI rats compared with sham-operated rats. In contrast, in Ang-(1-7)-treated rats, none of these parameters were significantly deteriorated, except the systolic dP/dt (Figure 1A and 1E). Coronary flow was measured *ex vivo* in a Langendorff setup. When compared with sham-operated hearts, baseline coronary flow was decreased in untreated MI rats, whereas in Ang-(1-7)-treated rats, baseline coronary flow was almost completely preserved (Figure 1F). Coronary endothelial function and maximal coronary flow were tested by a 2-minute infusion of 3 x 10⁻⁸ mol/L bradykinin and 10⁻⁵ mol/L adenosine, respectively. Bradykinin infusion evoked an increase in coronary flow in all 3 groups, but flow was still significantly lower in untreated infarcted hearts than in shamoperated hearts. Bradykinin-dependent flow in Ang-(1-7)- treated hearts was not significantly impaired. Maximal flow after adenosine infusion was significantly decreased in untreated MI rats compared with sham-operated rats, as well. In Ang-(1-7)-treated MI rats, the difference did not reach statistical significance (Table 2).

**Table 2.**

| ***Ex vivo* coronary flow** | | | |
|---|---|---|---|
| | **sham** | **MI control** | **MI Ang-(1-7)** |
| baseline | 8.7 ± 0.6 | 6.8 ± 0.3* | 8.2 ± 0.6 |
| bradykinin 3·10⁻⁸ M | 12.1 ± 0.6 | 9.9 ± 0.4* | 11.1 ± 0.7 |
| adenosine 10⁻⁵ M | 14.0 ± 0.5 | 10.8 ± 0.5* | 12.3 ± 0.8 |

| | | | |
|---|---|---|---|
| Results are expressed as mean ± SEM in ml/min per g ventricular weight, * p < 0.05. | | | |

### Endothelial Function

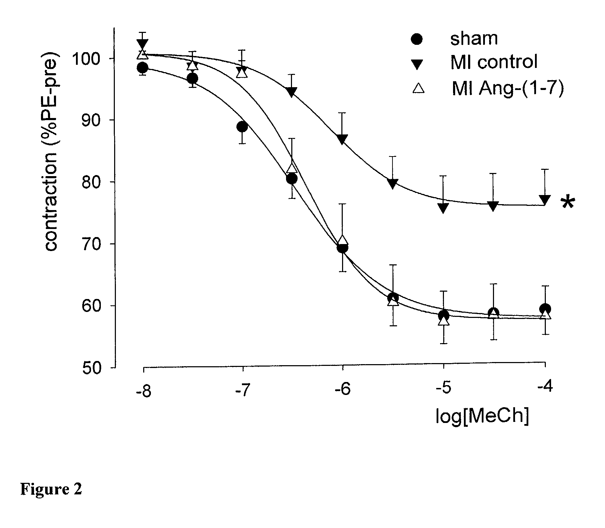

Endothelial dysfunction is a key feature in heart failure.¹⁴ To examine the effects of Ang-(1-7) treatment on this aspect of cardiac failure, we investigated endothelium-dependent relaxation in isolated aortic rings. Phenylephrine elicited similar contractile responses in all 3 groups (data not shown). The response to the endothelium-dependent vasodilator metacho- line was markedly decreased in rings from infarcted animals to 58.4% ±11.7% (P <0.05) of sham-operated rats. In Ang- (1-7)-treated rats, however, metacholine-induced relaxation was identical to sham rats (P <0.05 versus MI control; Figure 2). The relaxation in response to the endothelium independent vasodilator NaNO₂ (10⁻² mol/L) was equal in the 3 groups (data not shown).

### Discussion

In the present study, the effects of intravenous infusion of Ang-(1-7) on the development of cardiac function were examined in a rat coronary artery ligation model. We found that 8 weeks of Ang-(1-7) treatment prevented the deterioration of cardiac function, as shown by a 40% reduction in left ventricular end-diastolic pressure, an almost full preservation of coronary flow, and preserved aortic endothelial function. Although Ang-(1-7) has weak vasodilator activities,^{9,10} an increase in mean arterial pressure was found in the group infused with Ang-(1-7). Moreover, myocyte hypertrophy was attenuated by Ang-(1-7) infusion. Both results show an intracardiac mode of action of Ang-(1-7). This study shows that Ang-(1-7) is an effective agent in the attenuation of the development of heart failure after MI.

### Brief description of the drawings

**Figure 1.** Effects of myocardial infarction (MI) and angiotensin-(1-7) (Ang-(1-7)) treatment on hemodynamic parameters. LVP: left ventricular systolic pressure, LVEDP: left ventricular end-diastolic pressure, MAP: mean arterial pressure, +dP/dt and -dP/dt: maximal rate of increase and decrease of ventricular pressure, respectively, flow: baseline coronary flow; * p < 0.05 vs. sham.
**Figure 2.** Metacholine-dependent relaxation of phenylephrine (PE) precontracted aortic rings; * p < 0.05 vs. sham and p < 0.05 vs. Ang-(1-7).

### References

1. Pfeffer JM, Pfeffer MA, Mirsky I, et al. Regression of left ventricular hypertrophy and prevention of left ventricular dysfunction by captopril in the spontaneously hypertensive rat. *Proc Natl Acad Sci USA*. 1982;79: 3310-3314.
2. Swedberg K, Kjekshus J. Effects of enalapril on mortality in severe congestive heart failure: results of the Cooperative North Scandinavian Enalapril Survival Study (CONSENSUS). *Am J Cardiol.* 1988;62: 60A-66A.
3. Campbell DJ, Kladis A, Duncan A-M. Effects of converting enzyme inhibitors on angiotensin and bradykinin peptides. Hypertension 1994;23:439-449.
4. Ferrario CM, Chappell MC, Tallant EA, et al. Counterregulatory actions of angiotensin-(1-7). *Hypertension*. 1997;30:535-541.
5. Diez J, Panizo A, Hernandez M, Galindo MF, Cenarruzabeitir E, Parodo Mindan FJ. Quinapril inhibits c-Myc expression and normalizes smooth muscle cell proliferation in spontaneous hypertensive rats. Am J Hypertens 1997;10(Pt2):1147-1152.
6. Yamashita-J; Itoh-H; Ogawa-Y; Tamura-N; Takaya-K; Igaki-T; Doi-K; Chun-TH; Inoue-M; Masatsugu-K; Nakao-K Opposite regulation of Gax homeobox expression by angiotensin II and C-type natriuretic peptide. Hypertension. 1997(1Pt2):381-387.
7. Sonnenblick EH, Strobeck JE, Capasso JM, Factor SM. Ventricular hypertrophy: models and methods. In: Tarazi RC, Dunbar JB eds. *Perspectives in cardiovascular research*. New York: Raven Press, 1983; 8: 13-20.
8. Roks AJM, van Geel PP, Pinto YM, et al. Angiotensin-(1-7) is a modulator of the human renin-angiotensin system. *Hypertension.* 1999;34: 296-301.
9. Porsti I, Bara AT, Busse R, et al. Release of nitric oxide by angiotensin-(1-7) from porcine coronary endothelium: implications for a novel angiotensin receptor. *Br J Pharmacol*. 1994;111:652-654.
10. Brosnihan KB, Li P, Ferrario CM. Angiotensin-(1-7) dilates canine coronary arteries through kinins and nitric oxide. *Hypertension*. 1996;27: 523-528.
11. Mahon JM, Carr RD, Nicol AK, et al. Angiotensin(1-7) is an antagonist at the type 1 angiotensin II receptor. *J Hypertens*. 1994;12:1377-1381.
12. Kohara K, Brosnihan KB, Ferrario CM. Angiotensin(1-7) in the spontaneously hypertensive rat. *Peptides*. 1993;14:883-891.
13. Moriguchi A, Brosnihan KB, Kumagai H, et al. Mechanisms of hypertension in transgenic rats expressing the mouse Ren-2 gene. *Am J Physiol.* 1994;266:R1273-R1279.
14. Buikema H, Monnink SH, Tio RA, et al. Comparison of zofenopril and lisinopril to study the role of the sulfhydryl-group in improvement of endothelial dysfunction with ACE-inhibitors in experimental heart failure. *Br J Pharmacol*. 2000;130:1999-2007.
15. Capasso JM, Li P, Meggs LG, et al. Efficacy of angiotensin-converting enzyme inhibition and AT1 receptor blockade on cardiac pump performance after myocardial infarction in rats. *J Cardiovasc Pharmacol*. 1994;23:584-593.
16. Admiraal PJJ, Derkx FHM, Danser AHJ, et al. Metabolism and production of angiotensin I in different vascular beds in subjects with hypertension. *Hypertension.* 1990;15:44-55.

## Claims

1. Use of angiotensin-(1-7), or a functional part, derivative and/or analogue thereof, for the preparation of a medicament for enhancing cardiac function.

2. Use according to claim 1, wherein said cardiac function comprises left ventricular diastolic pressure, coronary flow, endothelial function and/or myocyte hypertrophy.

3. Use according to claim 2, wherein said endothelial function comprises aortic endothelial function and/or coronary endothelial function.

4. A pharmaceutical composition comprising angiotensin-(1-7), or a functional part, derivative and/or analogue thereof, and a carrier.

5. Use of angiotensin-(1-7), or a functional part, derivative and/or analogue thereof, for enhancing cardiac function.

6. Use according to claim 5, wherein said cardiac function comprises left ventricular diastolic pressure, coronary flow, endothelial function and/or myocyte hypertrophy.

7. Use according to claim 6, wherein said endothelial function comprises aortic endothelial function and/or coronary endothelial function.

8. A method for treating an animal suffering from, or at risk of suffering from, heart failure comprising administering to said animal a pharmaceutical composition according to claim 4.

9. A method according to claim 8, wherein said heart failure, or risk of heart failure, occurs after a myocardial infarction.

10. A method according to claim 8 or 9, wherein said pharmaceutical composition is administered to said animal by infusion.

11. Use of angiotensin-(1-7), or a functional part, derivative and/or analogue thereof, for treatment of an animal suffering from, or at risk of suffering from, heart failure.
